# EUROPÄISCHE PATENTSCHRIFT

(11) **EP 2 601 895 B1**
(45) Veröffentlichungstag und Bekanntmachung des Hinweises auf die Patenterteilung: **12.08.2015**
(21) Anmeldenummer: 12193321.2
(22) Anmeldetag: 20.11.2012
(51) Int. Cl.: A61B 17/29

(54) **Medizinisches Instrument**
Medical instrument
Instrument médical

(30) Priorität: 08.12.2011 DE 102011088003
(43) Veröffentlichungstag der Anmeldung: 12.06.2013
(73) Patentinhaber: Richard Wolf GmbH, 75438 Knittlingen (DE)
(72) Erfinder: Hofmann, Adrian, 75180 Pforzheim (DE)
(74) Vertreter: Patentanwälte Vollmann & Hemmer

(56) Entgegenhaltungen:
- US-A- 5 176 699
- US-B1- 6 425 906

## Beschreibung

Aus DE 665 208 sowie FR 2 688 681 sind medizinische Instrumente bekannt, bei denen ein axial bewegbares Betätigungselement mit zwei voneinander beabstandet angeordneten schwenkbaren Griffteilen derart bewegungsgekoppelt ist, dass das Betätigungselement durch das aufeinander zu Bewegen der Griffteile in eine erste Richtung verschoben wird und durch das voneinander weg Bewegen in eine zweite dazu entgegengesetzte Richtung verschoben wird.

Die Bewegungskopplung des Betätigungselements mit den Griffteilen erfolgt bei diesen Instrumenten dadurch, dass ein proximaler Endabschnitt des Betätigungselements als Zahnstange mit Verzahnungen an zwei voneinander abgewandten Längsseiten ausgebildet ist, wobei an jeder dieser Verzahnungen ein an den beiden Griffteilen zahnradförmig ausgebildeter Bereich im Eingriff ist. Durch den gleichzeitigen Eingriff der zahnradförmigen Bereiche der Griffteile in die auf Seiten des Betätigungselements ausgebildeten Verzahnungen sind die Schwenkbewegungen der beiden Griffteile synchronisiert. Bei den bekannten Instrumenten erweist es sich als nachteilig, dass deren Herstellung vergleichsweise aufwändig ist. Ein weiterer Nachteil dieser Instrumente besteht darin, dass es für eine Entnahme des Betätigungselements aus dem Instrument, beispielsweise zu Wartungszwecken, erforderlich ist, zuvor die Griffteile zu demontieren.

Weitere medizinische Instrumente sind aus der US-A-51766 99 und US-B-6 425 906 bekannt.

Vor diesem Hintergrund liegt der Erfindung die Aufgabe zugrunde, ein medizinisches Instrument der oben genannten Art zu schaffen, das gegenüber den bislang bekannten Instrumenten einen einfacheren Aufbau aufweist, und dessen Betätigungselement in einfacherer Weise demontiert und montiert werden kann.

Gelöst wird diese Aufgabe durch ein medizinisches Instrument mit den in Anspruch 1 angegebenen Merkmalen. Vorteilhafte Weiterbildungen dieses Instruments ergeben sich aus den Unteransprüchen, der nachfolgenden Beschreibung sowie der Zeichnung. Hierbei können gemäß der Erfindung die in den Unteransprüchen angegebenen Merkmale jeweils für sich, aber auch in geeigneter Kombination die erfindungsgemäße Lösung gemäß Anspruch 1 weiter ausgestalten.

Bei dem erfindungsgemäßen medizinischen Instrument handelt es sich bevorzugt um ein endoskopisches Schaftinstrument, beispielsweise um eine endoskopische Fasszange, Koagulationszange oder Schere. Das Instrument weist ein in einem Schaft axial verschiebbares Betätigungselement auf. Dieses vorzugsweise stangenförmige Betätigungselement kann distalseitig mit einem an dem distalen Ende des Instruments angeordneten Werkzeug und besonders vorteilhaft mit einer dort schwenkbeweglich angeordneten Zangen- oder Scherenbranche bewegungsgekoppelt sein, die zusammen mit einer an dem distalen Ende des Instruments feststehend bzw. starr angeordneten Zangen- oder Scherenbranche ein Zangen- bzw. Scherenmaul bildet.

Proximalseitig des Schaftes weist das erfindungsgemäße Instrument zwei beabstandet voneinander schwenkbar angelenkte Hebelarme auf. Jeder dieser Hebelarme ist Teil eines Griffteils zum Betätigen des Instruments. Gemäß der Erfindung sind die Hebelarme mit dem Betätigungselement und unmittelbar miteinander bewegungsgekoppelt. Das heißt, anders als bislang üblich sind die Griffteile nicht indirekt über das Betätigungselement miteinander bewegungsgekoppelt sondern direkt miteinander bewegungsgekoppelt, so dass die zur Synchronisierung der Schwenkbewegungen der Griffteile notwendige Bewegungskopplung der beiden Hebelarme dieser Griffteile miteinander unabhängig von der Bewegungskopplung der Hebelarme mit dem Betätigungselement erfolgt. Dies ermöglicht es, die Bewegungskopplung der Hebelarme mit dem Betätigungselement konstruktiv einfacher auszugestalten und erlaubt ferner einen fertigungstechnisch einfacheren Aufbau des Betätigungselements, an dem proximalseitig beispielsweise nur ein einfacher Mitnehmer ausgebildet sein kann, der in solcher Wirkverbindung mit den Hebelarmen stehen kann, dass eine Schwenkbewegung der direkt miteinander bewegungsgekoppelten Hebelarme eine Linearbewegung des Betätigungselements hervorruft. Weiter vorteilhaft schafft die von der Bewegungskopplung der beiden Hebelarme miteinander getrennte Bewegungskopplung der Hebelarme mit dem Betätigungselement die Möglichkeit, durch eine entsprechende Schwenkbewegung der Hebelarme, die außerhalb des zum Betätigen des Instruments erforderlichen Schwenkbereichs der Hebelarme liegt, die Bewegungskopplung der Hebelarme mit dem Betätigungselement zu lösen, während die unmittelbare Bewegungskopplung der Hebelarme miteinander bestehen bleibt. Auf diese Weise kann das Betätigungselement, falls erforderlich, in einfacher Weise von dem erfindungsgemäßen Instrument demontiert und anschließend wieder montiert werden.

Zur Schaffung der unmittelbaren Bewegungskopplung der beiden Hebelarme miteinander sind bevorzugt an einander zugewandten Seiten der Hebelarme aufeinander abrollende Wälzflächen ausgebildet. Bei dieser Ausgestaltung sind die einander kontaktierenden Wälzflächen an den Hebelarmen an einer der Schwenkachse des jeweils anderen Hebelarms zugewandten Außenseite angeordnet. Vorteilhaft sind die Wälzflächen kreisbogenförmig ausgebildet, wobei eine Mittelachse der Wälzflächen mit der Schwenkachse des jeweiligen Hebelarms übereinstimmt. Weiter vorteilhaft weisen die beiden kreisförmigen Wälzflächen einen gleich großen Radius auf, so dass keine Unter- bzw. Übersetzungbei der Bewegungskopplung der beiden Hebelarme stattfindet.

Grundsätzlich ist es möglich, dass die aufeinander abrollenden Wälzflächen der beiden Hebelarme eine Reibpaarung bilden, so dass die Bewegungskopplung der beiden Hebelarme miteinander kraftschlüssig erfolgt. Bevorzugt ist allerdings eine Ausgestaltung, bei der die Bewegungskopplung der beiden Hebelarme formschlüssig erfolgt. In diesem Zusammenhang ist vorteilhaft vorgesehen, dass die Wälzflächen der beiden Hebelarme verzahnt ausgebildet sind, wobei die an den beiden Wälzflächen ausgebildeten Verzahnungen miteinander in Eingriff sind.

Gemäß einer weiteren vorteilhaften Weiterbildung des erfindungsgemäßen Instruments ist das Betätigungselement vorzugsweise zwischen den Wälzflächen geführt. Die Führung des Betätigungselements ist hierbei derart, dass sich die Wälzflächen zumindest in einem Teilbereich immer kontaktieren und so eine unmittelbare Bewegungskopplung der beiden Hebelarme miteinander schaffen.

Zur Führung des Betätigungselements zwischen den Wälzflächen ist an den beiden Wälzflächen vorteilhaft jeweils außenseitig eine Nut ausgebildet. Die Nuten erstrecken sich jeweils als Kreisbogen über die Wälzflächen und sind vorzugsweise in der Schwenkebene der beiden Hebelarme oder in einer dazu parallelen Ebene angeordnet. Zusammen bilden die Nuten einen Aufnahmeraum für das Betätigungselement bzw. einen in Richtung quer zur Längsausdehnung des Betätigungselements geschlossenen Führungskanal.

Bevorzugt ist auch das Betätigungselement mit den beiden Hebelarmen über eine formschlüssige Verbindung bewegungsgekoppelt. Zu diesem Zweck kann an dem Betätigungselement vorteilhaft eine Erweiterung, vorzugsweise eine kugelförmige Erweiterung, ausgebildet sein, die in zwei an den Wälzflächen ausgebildete Ausnehmungen eingreift. Bei der an dem Betätigungselement ausgebildeten Erweiterung handelt es sich um einen vorzugsweise am proximalen Ende des Betätigungselements angeordneten Bereich, der gegenüber einem distalseitig daran anschließenden Abschnitt des Betätigungselements einen erweiterten Querschnitt aufweist. Die an den Wälzflächen der beiden Hebelarme vorgesehenen Ausnehmungen sind jeweils im Bereich der an den Wälzflächen ausgebildeten Nuten angeordnet, wobei eine in Längsrichtung der Wälzfläche verlaufende Mittelachse der Ausnehmung jeweils mit einer Mittelachse der Nut übereinstimmt. Wenn die beiden Wälzflächen durch ein gleichzeitiges Verschwenken der Hebelarme aufeinander abrollen, verändert sich die Position der an den Wälzflächen ausgebildeten Ausnehmungen, wodurch die in die beiden Ausnehmungen eingreifende Erweiterung und damit einhergehend das gesamte Betätigungselement in einem gewissen Bereich linear bewegt wird.

Bevorzugt sind die Wälzflächen in Richtung der Schwenkachsen der Hebelarme zweigeteilt ausgebildet, wobei eine an einem ersten Teil ausgebildete Verzahnung gegenüber einer an einem zweiten Teil ausgebildeten Verzahnung um eine Zahnlückenweite oder Zahndicke versetzt ausgebildet ist. Hierbei erfolgt die Zweiteilung der Wälzflächen zweckmäßigerweise in Richtung der Schwenkachse der Hebelarme. An den Umfangsabschnitten der Wälzflächen, an denen an dem ersten Teil der Wälzflächen Zähne ausgebildet sind, befinden sich an dem zweiten Teil der Wälzflächen Zahnlücken und an den Umfangsabschnitten der Wälzflächen, an denen an dem ersten Teil der Wälzflächen Zahnlücken ausgebildet sind, befinden sich an dem zweiten Teil der Wälzflächen Zähne. Hierbei weisen die an dem ersten Teil der Wälzflächen ausgebildete Verzahnung und die an dem zweiten Teil der Wälzflächen ausgebildete Verzahnung zweckmäßigerweise ein gleiches Zahn- und Zahnlückenprofil auf.

Die in den beiden Teilen der Wälzflächen zueinander versetzten Verzahnungen haben den Vorteil, dass zur Bildung der beiden Verzahnungen der Wälzfläche Verzahnungen mit einem vergleichsweise großen Modul verwendet werden können, so dass die Zahnlücken der einzelnen Verzahnungen verhältnismäßig groß sind und so einen großen Teil der Ausnehmungen zur Aufnahme der an dem Betätigungselement ausgebildeten Erweiterung bilden können. Gleichzeitig ermöglicht die versetzte Anordnung der Verzahnungen eine besonders spielfreie Bewegungskopplung der beiden Hebelarme miteinander.

Eine Ausgestaltung, bei der die Wälzflächen in einem zweiten Teil der Wälzflächen eine zu einem ersten Teil der Wälzflächen versetzte Verzahnung aufweist, lässt sich besonders einfach herstellen, wenn, wie es weiter bevorzugt vorgesehen ist, jeweils zwei Zahnradsegmente die Wälzfläche eines Hebelarms bilden, wobei alle Zahnradsegmente baugleich ausgebildet sind. D. h. das erfindungsgemäße Instrument weist insgesamt vier baugleiche Zahnradsegmente auf.

Zweckmäßigerweise weisen die Zahnradsegmente eine asymmetrische Verzahnung auf. Das heißt, die an einem Zahnradsegment ausgebildete Verzahnung beginnt mit einem Zahn und endet mit einer Zahnlücke. Zur Schaffung von zweigeteilten Wälzflächen, die in einem ersten Teil eine gegenüber dem zweiten Teil versetzt angeordnete Verzahnung aufweisen, sind zwei Zahnradsegmente übereinander anzuordnen, wobei ein zweites Zahnradsegment gegenüber einem ersten Zahnradsegment um 180° um eine in Radiusrichtung des Zahnradsegments verlaufende Achse verdreht ausgerichtet ist.

Es hat sich gezeigt, dass zur Verwirklichung der bei dem erfindungsgemäßen medizinischen Instrument erforderlichen axialen Verschiebewege des Betätigungselements Zahnradsegmente ausreichen, die mit zwei Zähnen und zwei Zahnlücken ausgestattet sind, wobei sich die an dem Zahnradsegment ausgebildete Verzahnung über einen Winkelbereich von 65° erstreckt. Insofern ist eine Ausgestaltung der Zahnradsegmente vorgesehen, bei der die Zahnradsegmente vorteilhaft jeweils zwei Zähne und zwei Zahnlücken aufweisen, wobei sie weiter vorteilhaft einen Winkel von 65° einschließen.

Bevorzugt sind die Zahnradsegmente in den Griffteilen zum Betätigen des erfindungsgemäßen Instruments integriert. So kann an jedem der beiden Griffteile eine Ausnehmung ausgebildet sein, in die jeweils zwei Zahnradsegmente übereinander in der oben beschriebenen Weise angeordnet sind. Des Weiteren können die beiden Zahnradsegmente eines Griffteils auch mit einem das Griffteil bildenden Kunststoff umspritzt sein.

Nachfolgend ist die Erfindung anhand eines in der Zeichnung dargestellten Ausführungsbeispiels näher erläutert. In der Zeichnung zeigt:
- Fig. 1: in einer schematisch vereinfachten perspektivischen Ansicht ein Bedienteil eines medizinischen Instruments,
- Fig. 2: das Bedienteil nach Fig. 1 in einer teilgeschnittenen Seitenansicht,
- Fig. 3: in einer Prinzipdarstellung, die Eingriffsverhältnisse von zwei Zahnradsegmenten und einem Betätigungselement in dem Bedienteil nach Fig. 1 in einer ersten Eingriffsstellung,
- Fig. 4: in einer Prinzipdarstellung, die Eingriffsverhältnisse von zwei Zahnradsegmenten und einem Betätigungselement in dem Bedienteil nach Fig. 1 in einer zweiten Eingriffsstellung,
- Fig. 5: in einer Prinzipdarstellung, die Eingriffsverhältnisse von zwei Zahnradsegmenten und einem Betätigungselement in dem Bedienteil nach Fig. 1 in einer dritten Eingriffsstellung,
- Fig. 6: die Eingriffsverhältnisse nach Fig. 3 in einer perspektivischen Darstellung,
- Fig. 7: die Eingriffsverhältnisse nach Fig. 4 in einer perspektivischen Darstellung,
- Fig. 8: die Eingriffsverhältnisse nach Fig. 5 in einer perspektivischen Darstellung und
- Fig. 9: ein Zahnradsegment in einer perspektivischen Einzeldarstellung.

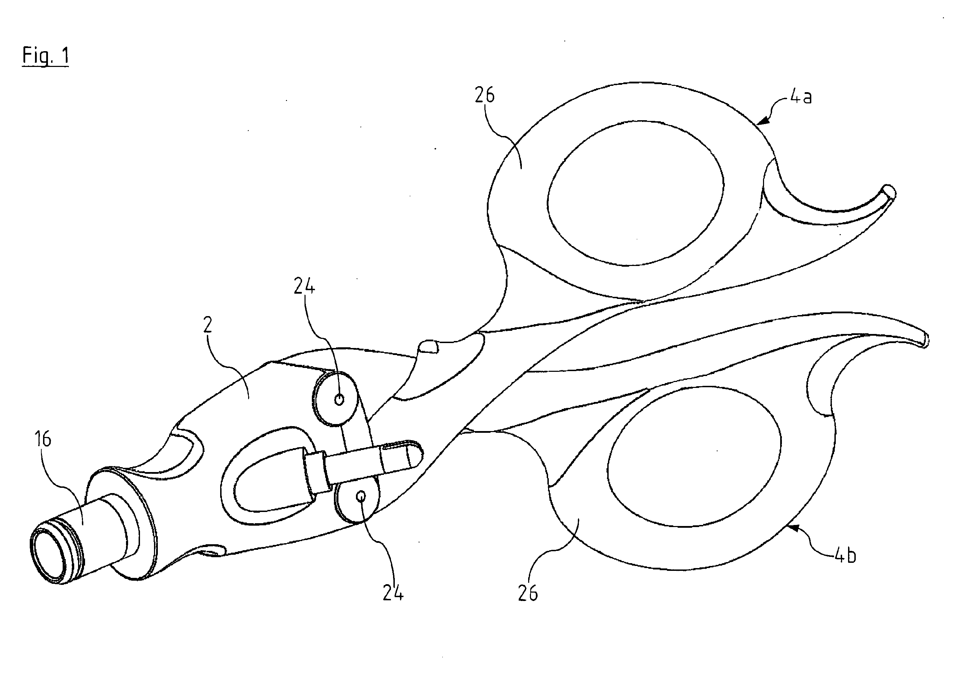

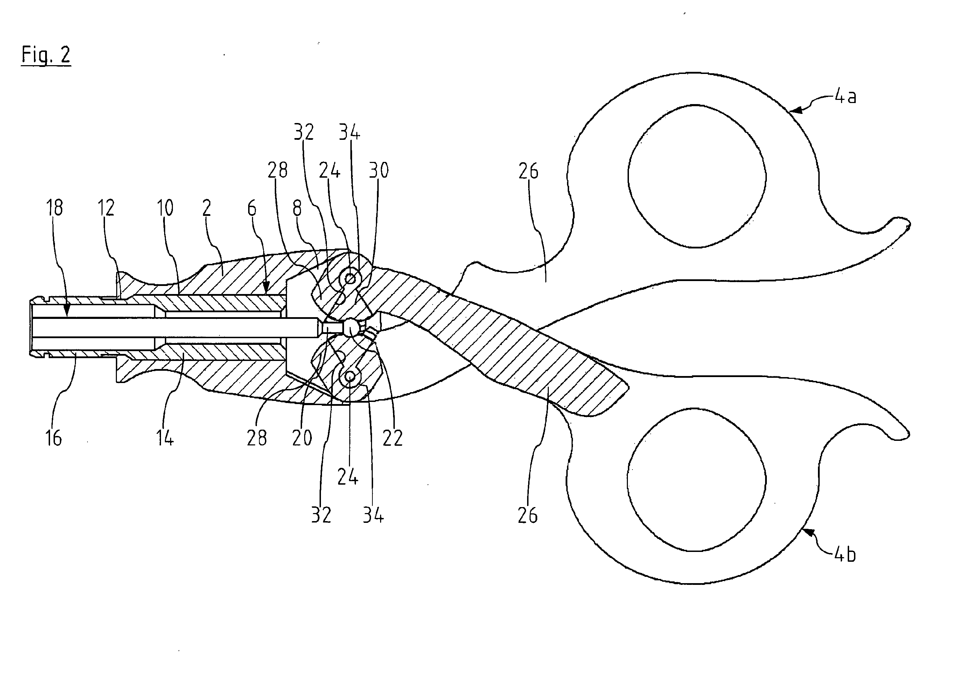

Die Figuren 1 und 2 zeigen ein Bedienteil eines medizinischen Instruments, welches das proximale Ende des Instruments bildet. Bei dem medizinischen Instrument handelt es sich um ein endoskopisches Schaftinstrument. Das Bedienteil weist ein Anschlussteil 2 auf, an dem zwei Griffteile 4a und 4b angelenkt sind.

Wie der Fig. 2 zu entnehmen ist, ist an dem Anschlussteil 2 eine Durchbrechung 6 ausgebildet, die sich in Längsrichtung des Anschlussteils 2 vollständig durch dieses hindurch erstreckt. Proximalseitig weist die Druchbrechung 6 einen Abschnitt 8 auf, der sich ausgehend von dem proximalen Ende des Anschlussteils 2 in distaler Richtung kontinuierlich verjüngt. An den Abschnitt 8 schließt sich distalseitig unter Bildung eines Absatzes ein zylindrischer Abschnitt 10 der Durchbrechung 6 an. Distalseitig des Abschnitts 10 verjüngt sich durch die Durchbrechung 6 konisch und geht in einen Abschnitt 12 über.

In der Durchbrechung 6 des Anschlussteils 2 ist in den Abschnitten 10 und 12 eine Hülse 14 angeordnet, wobei ein distaler Endabschnitt 16 der Hülse 14 distalseitig aus dem Anschlussteil 2 herausragt. Der Endabschnitt 16 dient zur Befestigung eines in der Zeichnung nicht dargestellten Schaftsrohrs des endoskopischen Schaftinstruments.

Durch dieses Schaftrohr sowie durch das Innenlumen der Hülse 14 ist ein Betätigungselement 18 in Form einer Zug-Druck-Stange geführt. Das Betätigungselement 18 ist in dem Schaftrohr sowie in dem Anschlussteil 2 axial verschiebbar. Aus der Zeichnung nicht ersichtlich erstreckt sich das Betätigungselement 18 durch das gesamte Schaftrohr und ist im Bereich des distalen Endes des Instruments mit einem dort beweglich angeordneten Werkzeug bewegungsgekoppelt.

An seinem proximalen Ende weist das Betätigungselement 18 einen Abschnitt 20 mit einem gegenüber dem übrigen Betätigungselement 18 verjüngten Querschnitt auf. Das proximale Ende des Betätigungselements 18 bildet eine kugelförmige Erweiterung 22, die sich an den Abschnitt 20 des Betätigungselements 18 anschließt. In dem Abschnitt 8 des Anschlussteils 2 sind in unmittelbarer Nähe zu dem proximalen Ende des Anschlussteils 2 zwei Gelenkstifte 24 beabstandet voneinander angeordnet. An diesen beiden Gelenkstiften 24 sind die Griffteile 4a und 4b schwenkbeweglich an dem Anschlussteil 2 angelenkt.

Die Griffteile 4a und 4b sind jeweils als zweiteilige Hebel ausgebildet. Sie weisen jeweils eine manuell zu greifenden Hebelarm 26 und einen Hebelarm 28 auf. Die Hebelarme 28 der Griffteile 4a und 4b sind jeweils im Wesentlichen in Richtung auf den Gelenkstift 24 ausgerichtet, an dem das jeweils andere Griffteil 4a bzw. 4b angelenkt ist. Insofern sind die Hebelarme 28 der beiden Griffteile 4a und 4b zwischen den Gelenkstiften 24 aufeinander zu ausgerichtet.

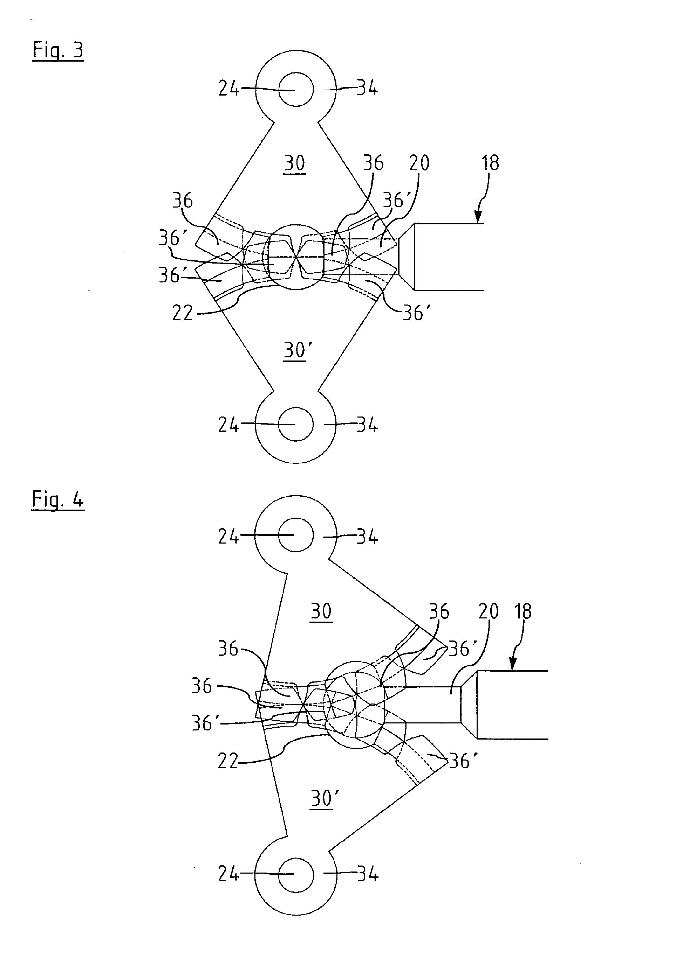

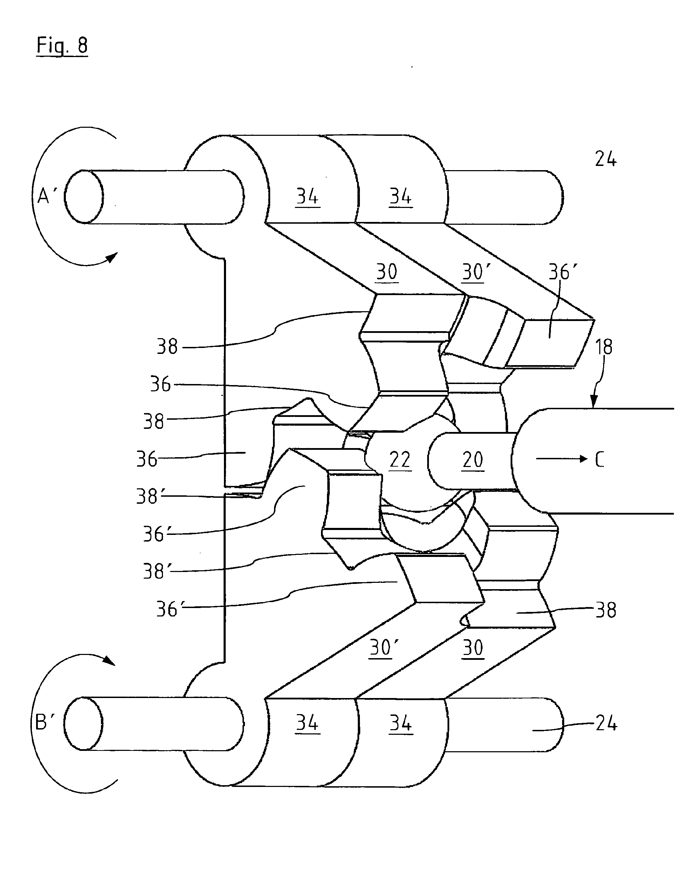

Die Hebelarme 28 der beiden Griffteile 4a und 4b sind unmittelbar miteinander bewegungsgekoppelt. Zu diesem Zweck weisen die Hebelarme 28 an den einander zugewandten Seiten jeweils eine Verzahnung auf, wobei die beiden Verzahnungen miteinander in Eingriff sind. Die Verzahnungen werden von Zahnradsegmenten 30 gebildet, die in Ausnehmungen 32 der Hebelarme 28 integriert sind. In Fig. 2 ist aus Gründen der besseren Übersichtlichkeit auf die Darstellung der Verzahnungen verzichtet worden. Diese werden aber aus den Fig. 3 - 9 deutlich.

Die Zahnradsegmente 30 haben im Wesentlichen die Form eines Kreissektors mit einer kreisbogenförmigen Außenseite, an der die Verzahnung ausgebildet ist und zwei von der kreisbogenförmigen Außenseite ausgehende spitz zueinander zulaufende gerade Außenseiten, an deren Enden ein Lagerauge 34 ausgebildet ist, das zur Aufnahme eines Gelenkstifts 24 dient. Die spitz aufeinander zulaufenden Außenseiten der Zahnradsegmente 30 schließen einen Winkel von 65° ein. Die Verzahnung der Zahnradsegmente 30 ist asymmetrisch und wird von zwei Zähnen 36 und zwei Zahnlücken 38 gebildet.

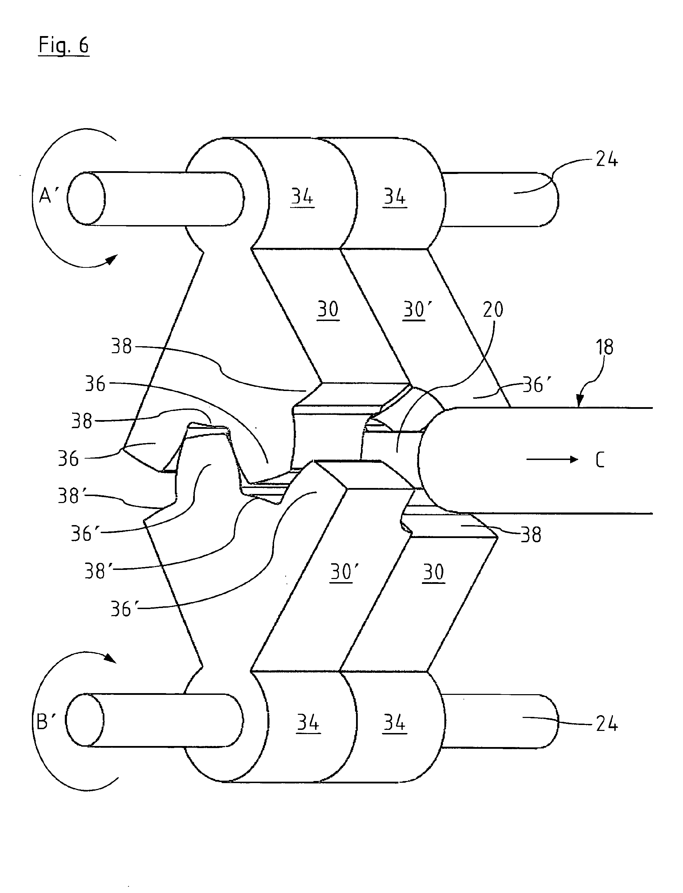

In den Ausnehmungen 32 der beiden Hebelarme 28 sind jeweils zwei Zahnradsegmente 30 übereinander angeordnet, wobei ein erstes Zahnradsegment 30 zu einem zweiten Zahnradsegment 30' um 180° gedreht angeordnet ist, sodass sich dort, wo sich eine Zahnlücke 38 des ersten Zahnradsegments 30 befindet, ein Zahn 36' des zweiten Zahnradsegments 30' angeordnet ist und dort, wo sich ein Zahn 36 des ersten Zahnradsegments 30 befindet, eine Zahnlücke 38' des zweiten Zahnradsegments 30' angeordnet ist, was insbesondere aus den Figuren 6 bis 8 deutlich wird. Die in den Hebelarmen 28 jeweils angeordneten Zahnradsegmente 30 und 3ß0' sind alle baugleich ausgebildet, also identisch

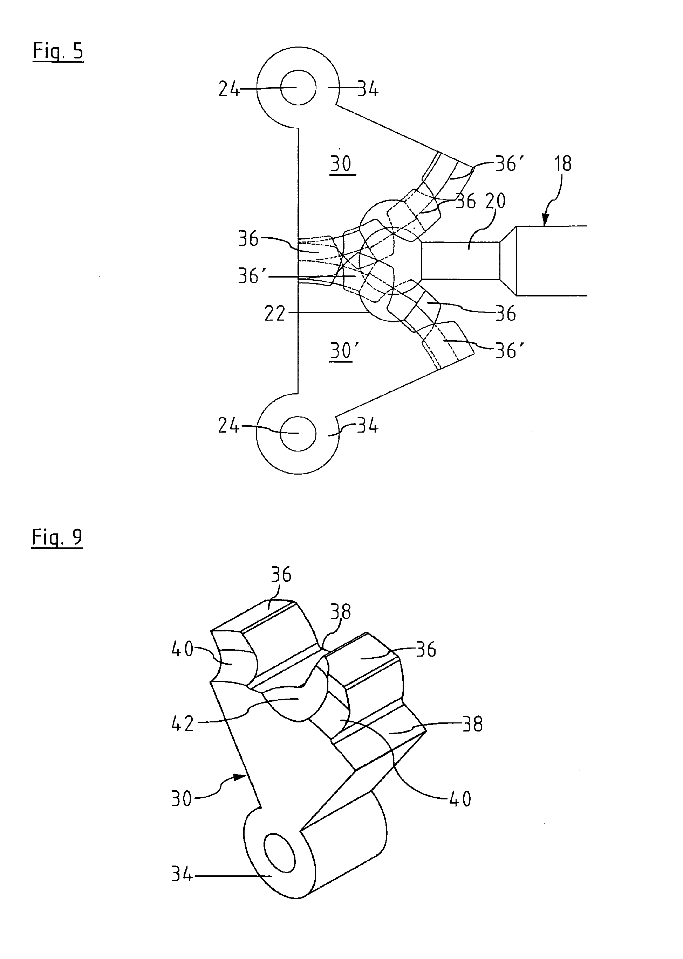

Die Hebelarme 28 sind über deren Zahnradsegmente 30 und 30' auch direkt mit dem Betätigungselement 18 bewegungsgekoppelt. Hierbei ist das Betätigungselement 18 zwischen den einander gegenüberliegend angeordneten und miteinander in Eingriff befindlichen Zahnradsegmenten 30 und 30' geführt. Zu diesem Zweck sind an den Zahnradsegmenten 30 und 30' an deren Zähnen 36 und 36' ausgehend von einer Flachseite der Zahnradsegmente 30 und 30' Ausnehmungen 40 ausgebildet, die sich im Wesentlichen über die gesamte Zahnhöhe der Zähne 36 und 36' erstrecken (Fig. 9). Bei zwei ineinander eingreifenden Zahnradsegmentpaaren der Hebelarme 28 bilden die an den Zahnradsegmenten 30 und 30' ausgebildeten Ausnehmungen 40 zusammen mit den Zahnlücken dieser Zahnradsegmente 30 und 30' einen Führungskanal für den Abschnitt 20 des Betätigungselements 18.

Zur Aufnahme der an dem proximalen Ende des Betätigungselements 18 ausgebildeten kugelförmigen Erweiterung 22 ist an jedem der Zahnradsegmente 30 und 30' eine weitere Ausnehmungen 42 ausgebildet, deren Innenkontur mit der Außenkontur der kugelförmigen Erweiterung 22 des Betätigungselements 18 korrespondiert. Zusammen bilden die Ausnehmungen 42 der übereinander angeordneten Zahnradsegmente 30 und 30' der Zahnradsegmentpaare der Hebel 28 einen im Wesentlichen kugelförmigen Aufnahmeraum, in dem die kugelförmige Erweiterung 22 des Betätigungselements 18 formschlüssig angeordnet ist.

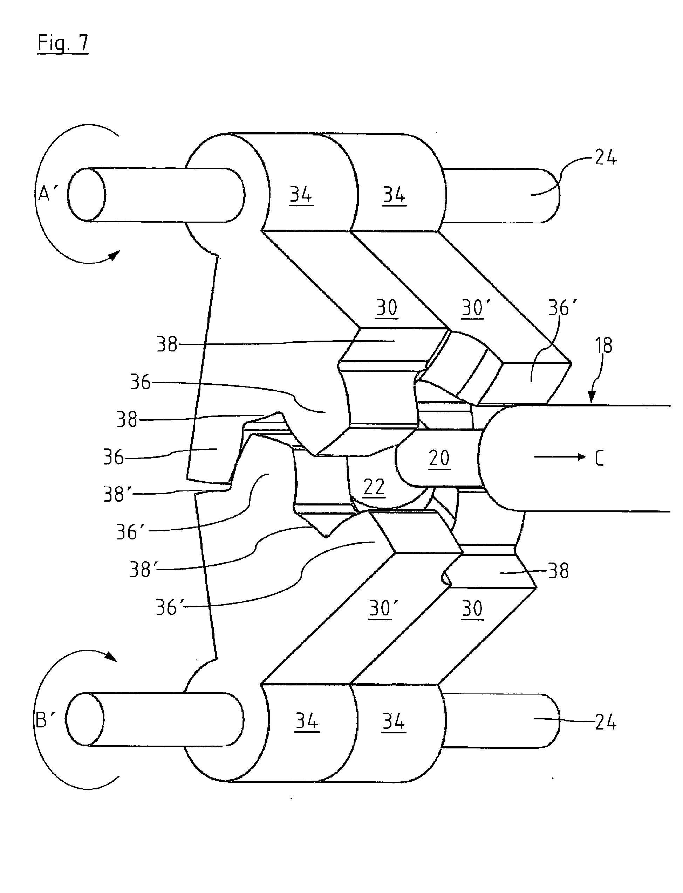

Die Funktionsweise des erfindungsgemäßen medizinischen Instruments wird insbesondere aus den Fig. 6 bis 8 deutlich. Ausgehend von einer in Fig. 6 gezeigten Ausgangssituation werden die Hebelarme 28 mit den darin jeweils integrierten Zahnradsegmenten 30 und 30' durch ein Verschwenken der Griffteile 4a und 4b aufeinander zu, so geschwenkt, dass sich das in den Fig. 6 bis 8 links dargestellte Zahnradsegmentpaar in Richtung A und das in den Fig. 6 bis 8 rechts dargestellte Zahnradsegmentpaar in Richtung B bewegt. Hierdurch bewegt sich der zwischen den vier Zahnradsegmenten 30 und 30' von den Ausnehmungen 42 dieser Zahnradsegmente 30 und 30' gebildete Ausnahmeraum, in der die kugelförmige Erweiterung 22 des Betätigungselements 18 formschlüssig angeordnet ist, in einem gewissen Maße linear in Richtung C, wodurch das Betätigungselement 18 in distaler Richtung verschoben wird (Fig. 7). Anschließend können die Griffteile 4a und 4b voneinander weg geschwenkt werden, wodurch das Betätigungselement 18 in umgekehrter Weise wieder in proximaler Richtung zurückgeschoben wird. Werden die Hebelarme ausgehend von dem in Fig. 7 dargestellten Zustand weiter vor verschwenkt, dass sich das in den Fig. 6 bis 8 links dargestellte Zahnsegmentpaar in Richtung A und das in den Fig. 6 bis 8 rechts dargestellte Zahnsegmentpaar in Richtung B bewegt, bewegen sich die Innenwandungen der an den Zahnradsegmenten 30 und 30' ausgebildeten Ausnehmungen 42 derart von der kugelförmigen Erweiterung 22 des Betätigungselements 18 weg, dass das Betätigungselement 18 von den Zahnradsegmenten 30 und 30' freigegeben wird und aus dem medizinischen Instrument entnommen werden kann.

### Bezugszeichenliste

- 2: Anschlussteil
- 4a, 4b: Griffteil
- 6: Durchbrechung
- 8: Abschnitt
- 10: Abschnitt
- 12: Abschnitt
- 14: Hülse
- 16: Endabschnitt
- 18: Betätigungselement
- 20: Abschnitt
- 22: Erweiterung
- 24: Gelenkstift
- 16: Hebelarm
- 28: Hebelarm
- 30, 30': Zahnradsegment
- 32: Ausnehmung
- 34: Lagerauge
- 36, 36': Zahn
- 38, 38': Zahnlücke
- 40: Ausnehmung
- 42: Ausnehmung

- A: Richtung
- B: Richtung
- C: Richtung

## Patentansprüche

1. Medizinisches Instrument, insbesondere endoskopisches Schaftinstrument, mit einem in einem Schaft axial verschiebbaren Betätigungselement (18) und mit zwei proximalseitig des Schafts beabstandet voneinander schwenkbar angelenkten Hebelarmen (28), die jeweils Teil eines Griffteils (4a, 4b) bilden, **dadurch gekennzeichnet, dass** die Hebelarme (28) mit dem Betätigungselement (18) und unmittelbar miteinander bewegungsgekoppelt sind.

2. Medizinisches Instrument nach Anspruch 1, bei dem an den einander zugewandten Seiten der Hebelarme (28) aufeinander abrollende Wälzflächen ausgebildet sind.

3. Medizinisches Instrument nach Anspruch 2, bei dem die Wälzflächen verzahnt ausgebildet sind.

4. Medizinisches Instrument nach einem der Ansprüche 2 oder 3, bei dem das Betätigungselement (18) zwischen den Wälzflächen geführt ist.

5. Medizinisches Instrument nach einem der Ansprüche 2 bis 4, bei dem an den Wälzflächen jeweils eine an der Außenseite der Wälzflächen angeordnete Nut ausgebildet ist.

6. Medizinisches Instrument nach einem der Ansprüche 2 bis 5, bei dem an dem Betätigungselement (18) eine vorzugsweise kugelförmige Erweiterung (22) ausgebildet ist, welche in zwei an den Wälzflächen ausgebildete Ausnehmungen eingreift.

7. Medizinisches Instrument nach einem der Ansprüche 2 bis 6, bei dem die Wälzflächen in Richtung der Schwenkachsen der Hebelarme (28) zweigeteilt ausgebildet sind, wobei eine an einem ersten Teil ausgebildete Verzahnung gegenüber einer an einem zweiten Teil ausgebildeten Verzahnung um eine Zahnlückenweite oder Zahndicke versetzt ausgebildet ist.

8. Medizinisches Instrument nach Anspruch 7, bei dem jeweils zwei Zahnradsegmente (30, 30') die Wälzflächen der Hebelarme (28) bilden, wobei alle Zahnradsegmente (30, 30') baugleich ausgebildet sind.

9. Medizinisches Instrument nach Anspruch 8, bei dem die Zahnradsegmente (30, 30') eine asymmetrische Verzahnung aufweisen.

10. Medizinisches Instrument nach einem der Ansprüche 8 oder 9, bei dem die Zahnradsegmente (30, 30') jeweils zwei Zähne (36, 36') und zwei Zahnlücken (38, 38') aufweist.

11. Medizinisches Instrument nach einem der Ansprüche 7 bis 9, bei dem die Zahnradsegmente (30, 30') einen Winkel von 65° einschließen.

12. Medizinisches Instrument, bei dem die Zahnradelemente (30, 30') in den Griffteilen (4a, 4b) integriert sind.

## Claims

1. A medical instrument, in particular endoscopic shank instrument, with an actuation element (18) which is axially displaceable in a shank and with two lever arms (28) which are pivotably articulated in a manner distanced to one another, at the proximal side of the shank and which in each case form part of a grip part (4a, 4b), **characterised in that** lever arms (28) are coupled in movement to the actuation element (18) and directly to one another.

2. A medical instrument according to claim 1, with which rolling surfaces rolling on one another are formed on the sides of the lever arms (28) which face one another.

3. A medical instrument according to claim 2, with which the rolling surfaces are designed in a toothed manner.

4. A medical instrument according to one of the claims 2 or 3, with which the actuation element (18) is led between the rolling surfaces.

5. A medical instrument according to one of the claims 2 to 4, with which in each case a groove arranged on the outer side of the rolling surfaces is formed on the rolling surfaces.

6. A medical instrument according to one of the claims 2 to 5, with which a preferably spherical widening (22) which engages into two recesses formed on the rolling surfaces, is formed on the actuation element (18).

7. A medical instrument according to one of the claims 2 to 6, with which the rolling surfaces are designed divided in two in the direction of the pivot axes of the lever arms (28), wherein a toothing formed on a first part is designed offset by a tooth gap width or tooth thickness, with respect to a toothing formed on a second part.

8. A medical instrument according to claim 7, with which in each case two cog segments (30, 30') form the rolling surfaces of the lever arms (28), wherein all cog segments (30, 30') are designed in a constructionally equal manner.

9. A medical instrument according to claim 8, with which the cog segments (30, 30') have an asymmetrical toothing.

10. A medical instrument according to one of the claims 8 or 9, with which the cog segments (30, 30') in each case comprise two teeth (36, 36') and two tooth gaps (38, 38').

11. A medical instrument according to one of the claims 7 to 9, with which the cog segments (30, 30') enclose and angle of 65°.

12. A medical instrument with which the cog segments (30, 30') are integrated in the grip parts (4a, 4b).

## Revendications

1. Instrument médical, en particulier instrument endoscopique en forme de tige, comprenant un élément d'actionnement (18) déplaçable axialement dans une tige et deux bras de levier (28) à distance l'un de l'autre du côté proximal de la tige et articulés de façon à pivoter, qui forment chacun une partie d'un élément de préhension (4a, 4b), **caractérisé en ce que** les bras de levier (28) sont couplés en mouvement avec l'élément d'actionnement (18) et directement l'un avec l'autre.

2. Instrument médical selon la revendication 1, dans lequel sont formées, sur les côtés des bras de levier (28) orientés l'un vers l'autre, des surfaces primitives de fonctionnement roulant l'une sur l'autre.

3. Instrument médical selon la revendication 2, dans lequel les surfaces primitives de fonctionnement sont pourvues d'une denture.

4. Instrument médical selon l'une des revendications 2 ou 3, dans lequel l'élément d'actionnement (18) est guidé entre les surfaces primitives de fonctionnement.

5. Instrument médical selon l'une des revendications 2 à 4, dans lequel est formée, sur chaque surface primitive de fonctionnement, une rainure disposée sur la face externe des surfaces primitives de fonctionnement.

6. Instrument médical selon l'une des revendications 2 à 5, dans lequel est formée au niveau du dispositif d'actionnement (18) une extension (22), de préférence de forme conique, qui s'engage dans deux évidements réalisés sur les surfaces primitives de fonctionnement.

7. Instrument médical selon l'une des revendications 2 à 6, dans lequel les surfaces primitives de fonctionnement sont réalisées en deux parties dans la direction des axes de pivotement des bras de levier (28), une denture formée sur une première partie étant réalisée de manière à être décalée d'une largeur d'entredent ou d'une épaisseur de dent par rapport à une denture formée sur une seconde partie.

8. Instrument médical selon la revendication 7, dans lequel respectivement deux segments de roue dentée (30, 30') forment les surfaces primitives de fonctionnement des bras de levier (28), tous les segments de roue dentée (30, 30') étant réalisés de manière identique.

9. Instrument médical selon la revendication 8, dans lequel les segments de roue dentée (30, 30') ont une denture asymétrique.

10. Instrument médical selon l'une des revendications 8 ou 9, dans lequel les segments de roue dentée (30, 30') présentent chacun deux dents (36, 36') et deux entredents (38, 38').

11. Instrument médical selon l'une des revendications 7 à 9, dans lequel les segments de roue dentée (30, 30') forment un angle de 65°.

12. Instrument médical dans lequel les éléments de roue dentée (30, 30') sont intégrés dans les éléments de préhension (4a, 4b).
